(19) Europäisches Patentamt
European Patent Office
Office européen des brevets

(11) **EP 4 115 216 B1**

(12) **EUROPEAN PATENT SPECIFICATION**

(45) Date of publication and mention
of the grant of the patent:
**08.04.2026 Bulletin 2026/15**

(21) Application number: **21765294.0**

(22) Date of filing: **18.02.2021**

(51) International Patent Classification (IPC):
*G02B 5/00* (2006.01)    *G02C 7/04* (2006.01)
*G02C 7/16* (2006.01)

(52) Cooperative Patent Classification (CPC):
**G02C 7/16; G02C 7/046;** G02C 2202/24

(86) International application number:
**PCT/AU2021/050137**

(87) International publication number:
**WO 2021/174289 (10.09.2021 Gazette 2021/36)**

(54) **CONTACT LENS APPARATUS FOR MYOPIA MANAGEMENT**

KONTAKTLINSENVORRICHTUNG FÜR MYOPIE-MANAGEMENT

APPAREIL À LENTILLE DE CONTACT POUR LA GESTION DE LA MYOPIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.03.2020 AU 2020900607**

(43) Date of publication of application:
**11.01.2023 Bulletin 2023/02**

(73) Proprietor: **Nthalmic Holding Pty Ltd
Sydney NSW 2019 (AU)**

(72) Inventors:
• **BAKARAJU, Ravi Chandra
Sydney, New South Wales 2019 (AU)**
• **EHRMANN, Klaus
Sydney, New South Wales 2019 (AU)**

(74) Representative: **Kurig, Thomas
Becker Kurig & Partner
Patentanwälte mbB
Bavariastraße 7
80336 München (DE)**

(56) References cited:
EP-A1- 2 168 534          WO-A1-2012/102332
WO-A1-2013/082545    GB-A- 1 340 877
US-A- 5 260 727          US-A- 5 965 330
US-A1- 2006 034 003    US-A1- 2017 146 820
US-A1- 2019 314 146

• **DANIEL IAN FLITCROFT; ELISE N HARB;
CHRISTINE FRANCES WILDSOET: "The Spatial
Frequency Content of Urban and Indoor
Environments as a Potential Risk Factor for
Myopia Development", INVESTIGATIVE
OPHTHALMOLOGY & VISUAL SCIENCE, vol. 60,
no. 9, 1 July 2019 (2019-07-01), pages 6452,
XP002810915, Retrieved from the Internet
<URL:https://iovs.arvojournals.org/article.aspx?
articleid=2747052> [retrieved on 20240130]**
• **TORRALBA ANTONIO ET AL: "Statistics of
natural image categories", vol. 14, no. 3, 12 May
2003 (2003-05-12), GB, pages 391 - 412,
XP093125121, ISSN: 0954-898X, Retrieved from
the Internet <URL:https://web.mit.edu/torralba/
www/ne3302.pdf> DOI: 10.1088/
0954-898X_14_3_302**

**Description**

CROSS REFERENCE

This application claims priority to Australian Provisional Application Serial No. 2020/900607 filed on March 01, 2020, entitled "Contact lens apparatus".

FIELD OF THE INVENTION

The present disclosure particularly relates to contact lens apparatus and/or methods for myopia management. The present disclosure is directed towards modifying the incoming light through contact lenses that utilises a stop signal to decelerate the rate of myopia progression.

BACKGROUND OF THE INVENTION

Human eyes are hyperopic at birth, where the length of the eyeball is too short for the total optical power of the eye. As the person ages from birth to adolescence, the eyeball continues to grow until the eye's refractive state stabilises. The growth of the eye is controlled by a feedback mechanism and regulated predominantly by the visual experience, to match the eye's optics with the eye length and maintain homeostasis. This process is referred to as emmetropisation.

The signals that guide the emmetropisation process are initiated at the retina level. The retinal image characteristics are monitored by a biological process that modulate the signal to start or stop, accelerate, or slow eye growth. Derailing from the emmetropisation process results in refractive disorders like myopia. The problem of myopia is rapidly increasing, and it is predicted that half of the world's population may be myopic by 2050.

A simple pair of standard single vision contact lenses can correct myopia. Although such devices can correct the refractive error, they do not address the underlying cause of the excessive eye growth which is associated with debilitating vision threatening conditions. There remains a need for contact lenses that not only correct the underlying refractive error but also prevent excessive eye lengthening.

Almost all the contact lens design options available for retarding the rate of myopia progression, include some form of optical manipulation of the retinal image characteristics, for example, simultaneous defocus, positive spherical aberration, positive power in the centre and/or periphery of the optic zone, or manipulation of higher order aberrations. One of the weaknesses of such optical designs is that they compromise the quality of vision.

Given the influence of compliance of ophthalmic lens wear on the efficacy, significant reduction of visual performance may promote poor compliance, thus resulting in poorer efficacy for retarding the rate of myopia progression. Accordingly, what is needed are optical designs for the correction of myopia and retardation of progression, which do not cause the visual disturbances associated with manipulation of optical power.

GB 1 340 877 A discloses a contact lens including a light-transmitting area, provided with correction for distance vision, centrally located, and a surrounding opaque region defining an essentially circular pattern, having light-transmitting regions communicating with area and extending radially from this area, these regions having a width substantially smaller than the diameter of area.

WO 2012/102332 A1 discloses a presbyopia correction body including a transparent thin plate, wherein the transparent thin plate has an opaque part and a transparent diaphragm part formed in the opaque part. The transparent thin plate includes a lens or a film. The presbyopia correction body is to be worn on the surface of a cornea in one or corneas in both eyes, and the presbyopia correction body can be focused through the transparent diaphragm part.

Embodiments of the present disclosure are directed towards alternative methods devices or means to retard the progression of myopia that do not utilise any kind of myopic defocus, whether on- or off-axis, or any positive spherical aberration, or any kind of optical blur as a stop signal.

DEFINITIONS

Terms are used herein as generally used by a person skilled in the art, unless otherwise defined in the following. The term "myopic eye" means an eye that is either already experiencing myopia, is in the stage of pre-myopia, or is diagnosed to have a refractive condition that is progressing towards myopia.

The term "progressing myopic eye" means an eye with established myopia that is diagnosed to be progressing, as gauged by either the change in refractive error of at least -0.25 D/year or the change in axial length of at least 0.1 mm/year.

The term "pre myopic eye" or "an eye at risk of becoming myopic" means an eye, which could be emmetropic or is low hyperopic at the time but has been identified to have an increased risk of becoming myopic based on genetic factors (e.g., both parents are myopic) and/or age (e.g., being low hyperopic at a young age) and/or environmental factors (e.g.,

time spent outdoors) and/or behavioural factors (e.g., time spent performing near tasks).

The term "optical stop signal" means a signal that may facilitate slowing, reversing, arresting, retarding, inhibiting, or controlling the growth of an eye and/or refractive condition of the eye.

The term "contact lens" means a finished soft, hard or a hybrid contact lens to be fitted on the cornea of a wearer to affect the optical performance of the eye, usually packaged in a vial, blister pack or similar. The term "orthokeratology" lens may mean a contact lens used to flatten the cornea to provide vision correction.

The term "optical zone" means the region on the contact lens which has the prescribed optical effect. The term "optical centre of the contact lens" means the geometric centre of the optical zone of the contact lens. The terms geometrical and geometric centre are essentially the same.

The term or phrase "single vision optical zone" or "substantially single vision optics or profile" may mean that the optical zone has a uniform power distribution with or without asphericity in the power variation across the optic zone. The single vision optical zone may be further classified to include toric or astigmatic component to correct the distance refractive error.

## SUMMARY OF THE INVENTION

The present invention is provided in the appended claims. The following disclosure serves as a better understanding of the present invention. Certain embodiments of the present disclosure include contact lenses, both soft and rigid, including orthokeratology, and/or methods for altering the properties of incoming light entering a human eye. Certain embodiments of the present disclosure are directed to the configuration of contact lenses, methods and/or systems for correcting and treating refractive errors.

Certain embodiments of the present disclosure are aimed to both correct the myopic refractive error and simultaneously provide an optical signal that discourages further eye growth or progression of myopia. Certain embodiments of the present disclosure particularly relates to contact lens apparatus and/or methods for myopia management. The present disclosure is directed towards modifying the incoming light through contact lenses that utilise an optical stop signal to decelerate the rate of myopia progression.

More specifically, the present disclosure relates to a contact lens that is purposefully configured with a non-circular non-transparent region serving as an aperture stop, over an otherwise substantially single vision optic zone, that may facilitate altered point spread functions and modulation transfer functions on the retina; wherein the altered point spread functions and modulation transfer functions on the retina may further be characterised by a spectral signature of the retinal image; wherein the light energy within the spectral signature is purposefully redistributed in the oblique frequencies; which may serve as an optical stop signal for inhibiting, reducing, or controlling progressive myopic refractive error.

The present invention relates to a device, system and/or method for reducing the rate of myopia progression, and particularly to an advanced contact lens configured with a non-circular non-transparent region serving as an aperture stop within the optic zone of the lens. The present invention relates to a device, system and/or method for altering the retinal image characteristics by utilising or introducing non-circular non-transparent region serving as an aperture stop on a soft or rigid contact lens. The resulting image formed on the retina, when incoming light bundle passes through an eye and the contact lens of the present disclosure, comprises of additional features in the oblique frequencies of the retinal image, or have the energy redistributed into the oblique frequencies of the retinal image. As a result, the altered retinal imagery wherein the incoming light energy bundle is redistributed to oblique frequencies may provide a stop signal for the growth of a myopic eye.

Certain embodiments of the disclosure, the contemplated features of the effective non-circular non-transparent region, serving as an aperture stop, may be introduced on a rigid or a soft contact lens, wherein in the aperture stop may be configured, at least in part, on the front surface, back surface, or embedded within in the matrix of the contact lens. In some examples, wherein the aperture stop is configured on the front or back surface of the contact lenses, traditional or conventional printing technologies often used in cosmetic contact lens industry may be adapted. In other examples, wherein the non-circular non-transparent aperture is configured within the matrix of the material, laser-etching or engraving approaches may be contemplated.

Certain embodiments of this disclosure contemplates various exemplary methods that are directed to the purposeful redistribution of energy into oblique frequencies of the image formed on the retina of a myopic eye. The current disclosure postulates that by redistributing energy into oblique frequencies, the characteristics of the spectral signature of the retinal image formed utilising the methods and devices of the current disclosure mimic the retinal images obtained by viewing natural scenes, which in-turn may produce an optical stop signal for reducing the rate of myopia progression. The proposed disclosure utilises a contact lens that is purposefully designed with non-circular non-transparent region, serving as an aperture stop, over essentially a single vision optical zone, to purposefully redistribute, at least in part, the incoming light energy bundle into the oblique orientations within the spectra signature of the image, formed on the central and/or peripheral retina.

Certain embodiments of this disclosure describes contact lenses for at least one of slowing, retarding or preventing myopia progression, wherein the contact lens comprises of an optical zone with an optical centre, and a non-optical zone; wherein the optical zone comprises, at least the following: (i) a transparent region with a substantially single vision power profile configured to substantially match the refractive error of the myopic eye providing an in-focus retinal image of an incoming light bundle entering the myopic eye; wherein the retinal image when characterised using a point spread function, or an optical transfer function or a modulation transfer function or a convolution image simulation operation results in a spectral signature; and (ii) a non-circular non-transparent region circumscribing the transparent region configured to form an aperture stop of the contact lens; wherein the non-circular non-transparent aperture stop of the contact lens is capable of, at least in part, redistributing the incoming light into a plurality of oblique spatial frequencies of the spectral signature such that the redistributed spectral image mimics a spectral signature of an image formed when viewing a natural scene; wherein the redistributed spectral image is substantially different from a spectral signature of a man-made scene which may be predominantly dominated with information in the horizontal and vertical frequencies; wherein the redistributed spectral image on the retina of the myopic eye provides an optical stop signal to slow the progression of the myopic eye.

According to the claimed invention, the non-circular non-transparent region, serving as an aperture stop, spans an area of at least 2.5 square millimetres, at least 5 square millimetres, at least 7.5 square millimetres, at least 10 square millimetres or at least 12.5 square millimetres. According to the claimed invention, the transparent region with a substantially single vision power profile, spans an area of at least 12.5 square millimetres.

In some embodiments, the non-circular non-transparent region, serving as an aperture stop may be decentred from the optic centre of the contact lens and the magnitude of decentration may be approximately at least 0.125 mm, at least 0.25 mm, at least 0.5 mm, at least 0.75 mm or at least 1 mm. In some embodiments, the direction of decentration of the non-circular non-transparent region may be in the horizontal, vertical, superior, or inferior direction, while in yet some other embodiments, the direction of decentration of the non-circular non-transparent region may be in the oblique directions.

In other embodiments, the non-circular non-transparent region, serving as an aperture stop, may be shaped substantially like a regular polygon between 3 and 12 sides, between 5 and 12 sides, between 5 and 8 sides, between 5 and 10 sides, or between 6 and 14 sides. In yet another embodiment, the non-circular non-transparent region may specifically exclude the shape of a rectangle, a square or a rhombus. In other embodiments, where the non-circular non-transparent region includes the shape of a rectangle, a square, or a rhombus, the non-circular non-transparent region may need to be oriented in such a manner that the rectangle, the square or the rhombus may need to be positioned at an oblique angle. In some embodiments appropriate orientation or stabilisation systems of the contact lens may be considered to position the lenses at a desired oblique angle.

In accordance with another embodiment of the present disclosure, the aperture stop may be triangular, pentagonal, hexagonal, heptagonal, octagonal, or star shaped. In accordance with other embodiments of the present disclosure, the boundaries defining the non-circular non-transparent aperture stop may be configured with non-straight lines, e.g., curvilinear lines.

In other embodiments, the non-circular non-transparent region, serving as an aperture stop, may be configured translucent, or partially opaque, or substantially opaque. To facilitate the incorporation of translucent, or partially opaque, or substantially opaque non-circular non-transparent regions within the contact lens, traditional tools of trade in the cosmetic industry may be considered.

In yet another embodiment the non-circular non-transparent region, serving as an aperture stop, is configured such that its diameter substantially larger than the physiological pupil of the myopic eye, measured in a scotopic, a mesopic, or a dimly-lit lighting condition.

In certain embodiments of this disclosure, the transparent region may include spherical power and/or astigmatic power; while in some other embodiments, the transparent region may be further configured with additional positive or negative spherical aberration to optimise the visual performance for the myopic eye. In certain embodiments, the image scenes used for comparison and/or evaluation of the performance of the spectral signature of the retinal image formed by one or more embodiments of the disclosure may include various images representing natural scenes. For example, a forest scene, a mountain scene, a field scene, a beach scene, a coast scene, a river scene, or a waterfall scene.

In certain embodiments, the image scenes used for comparison and/or evaluation of the performance of the spectral signature of the retinal image formed by one or more embodiments of the disclosure may include various images representing man-made scenes. For example, an indoor scene, a street scene, a high-building scene, a city-view scene, a highway scene, an office scene, or a portrait scene.

In some embodiments, the contact lens of the disclosure is configured such that it capable of providing the wearer with adequate visual field that may be substantially indistinguishable from a conventional or traditional single vision contact lens that is otherwise free of the non-circular non-transparent region of the current disclosure. In other embodiments, the lens may be configured such that it is capable of providing the wearer with adequate visual performance that is substantially indistinguishable from a conventional or traditional single vision contact lens that is substantially free of the non-circular non-transparent region of the current disclosure.

In another embodiment, the lens may be configured such that it is capable of providing visible light transmittance that is within at least 80%, at least 85%, at least 90%, or at least 95% of the visible light transmittance provided by a conventional or traditional single vision contact lens that is free from the non-circular non-transparent region of the current disclosure.

In some embodiments, the optical performance evaluation of the contact lens examples of the disclosure may include: (i) use of at least one pupil diameter between 3 mm and 6 mm, inclusive; (ii) use of at least one visible light wavelength between 460 nm to 760 nm, inclusive; (iii) use of at least one field angle between 0 and 30 degrees, inclusive; (iv) use of at least one of the image quality metrics including a point spread function, an optical transfer function, a modulation transfer function, or a simulation of the retinal image using standard convolution operations, resulting in the computation of a spectral signature of the retinal image; and (v) use of schematic or physical or bench-top model eyes.

In some examples, the performance of one or more embodiments of the disclosure may be determined by comparing the spectral signature of the retinal image formed by employing one or more embodiments of the disclosure on a schematic, physical or bench-top model eye with the spectral signatures of the representative images of a natural scene, or a man-made scene.

In accordance with one of the embodiments, the present disclosure is directed to contact lenses configured with design features, for example, a non-circular non-transparent aperture stop within a substantially single vision optic zone, that may overcome the limitations of the prior art by providing a retinal image profile that is akin to a retinal image formed when viewing an object from a natural scene.

In accordance with one of the embodiments, the present disclosure is directed to contact lenses configured with design features, for example, a non-circular non-transparent aperture stop within a substantially single vision optic zone, that may overcome the limitations of the prior art by providing a retinal image profile that is substantially different to a retinal image formed when viewing an object from a man-made scene.

In another embodiment, the present invention is directed to an orthokeratology lens for at least one of slowing, retarding, or preventing myopia progression. In one embodiment, the said orthokeratology lens comprising a front surface, a back surface, a non-circular back optic zone, an optical centre; the non-circular back optical zone around the optic centre is configured such that the flattening of the corneal surface provides, at least in part, adequate foveal correction, and further configures a non-circular quasi aperture to provide, at least in part, an increase in energy distributed in the oblique orientations within the spectral signature of the retinal image thus providing a stop signal to reduce the rate of myopia progression.

Certain embodiments of the present disclosure also relate to an orthokeratology contact lens configured to effectively result in a non-circular effective optical quasi-aperture within the treatment zone, to correct the distance refractive error of the wearer. Wherein, the effective non-circular quasi-aperture alters the transmitted properties of the incoming light such that, the incoming light energy is redistributed into oblique frequencies of the spectral signature mimicking the spectral signature of images formed when viewing natural scenes. In other embodiments relating to the said orthokeratology contact lens, the back surface of the contact lens may be configured to effectively result in an elliptical effective quasi-aperture with the treatment zone to correct the distance refractive error of the wearer may be positioned obliquely, for example, the position of the elliptical effective quasi aperture may be between 25 and 75 degrees, or between 110 and 160 degrees; wherein the effective non-circular quasi-aperture positioned in an oblique orientation is configured such that it purposefully alters the transmitted properties of the incoming light such that, the energy is redistributed into a plurality of oblique frequencies of the spectral signature.

The spectral signature of the retinal image with redistributed energy into oblique frequencies mimics the spectral signature of an image obtained when viewing natural viewing scenes. The current disclosure postulates that experiencing retinal images that are akin to natural scenes which have oblique frequency content has a protective effect against progressive myopia. To facilitate such a treatment with an orthokeratology lens capable of providing a rotated elliptical quasi-optical aperture on the cornea may require a dedication stabilisation zone incorporated within the at least one peripheral zone of the orthokeratology lens.

The embodiments presented in this invention disclosure are directed to the ongoing need for enhanced ophthalmic lenses that may inhibit or reduce the rate of progression of myopia, while providing adequate vision performance to the wearer. Various aspects of the embodiments of the present invention disclosure address such needs of a wearer.

BRIEF DESCRIPTION OF THE FIGURES

Figure 1 illustrates spectral signatures of two distinct types of visual scenes, a representative sample for a natural scene and another representative sample of a man-made scene, as disclosed herein.

Figure 2 illustrates spectral signatures of fourteen (14) different types of visual scenes, a representative sample of seven (7) natural scenes and seven (7) man-made scenes, as disclosed herein.

Figure 3 illustrates the single-pass point spread function at the retina, when collimated light passes through a circular, elliptical, triangular, and square apertures of an eye as an optical system, as disclosed herein.

Figure 4 illustrates the single-pass point spread function at the retina, when collimated light passes through a pentagonal, hexagonal, octagonal and irregular hexagon shaped apertures of an eye as an optical system, as disclosed herein.

Figure 5 illustrates outlines/sketches of two man-made scenes, namely, (i) a representation of a city-skyline; and (ii) a representation of an indoor office setting.

Figure 6 frontal view and cross-sectional view of an exemplary contact lens embodiment, not to scale, as disclosed herein. The non-circular non-transparent optical aperture stop comprises a triangular shaped transparent region within the optic zone.

Figure 7 illustrates the spectral signatures of the retinal images formed by one of the exemplary embodiments disclosed in Figure 6 fitted on a schematic myopic model eye. The two spectral signatures are obtained in two different configurations, (a) with, and (b) without, the non-circular non-transparent aperture stop features, as disclosed herein.

Figure 8 frontal view and cross-sectional view of an exemplary contact lens embodiment, not to scale, as disclosed herein. The non-circular non-transparent optical aperture stop comprises a regular hexagon shaped transparent region within the optic zone.

Figure 9 illustrates the spectral signatures of the retinal images formed by one of the exemplary embodiments disclosed in Figure 8 fitted on a schematic myopic model eye. The two spectral signatures are obtained in two different configurations, (a) with, and (b) without, the non-circular non-transparent aperture stop features, as disclosed herein.

Figure 10 frontal view and cross-sectional view of an exemplary contact lens embodiment, not to scale, as disclosed herein. The non-circular non-transparent optical aperture stop comprises a star-shaped shaped transparent region within the optic zone.

Figure 11 illustrates the spectral signatures of the retinal images formed by one of the exemplary embodiments disclosed in Figure 10 fitted on a schematic myopic model eye. The two spectral signatures are obtained in two different configurations, (a) with, and (b) without, the non-circular non-transparent aperture stop features, as disclosed herein.

Figure 12 frontal view and cross-sectional view of an exemplary contact lens embodiment, not to scale, as disclosed herein. The non-circular non-transparent optical aperture stop comprises a Maltese-cross shaped transparent region within the optic zone.

Figure 13 illustrates the spectral signatures of the retinal images formed by one of the exemplary embodiments disclosed in Figure 12 fitted on a schematic myopic model eye. The two spectral signatures are obtained in two different configurations, (a) with, and (b) without, the non-circular non-transparent aperture stop features, as disclosed herein.

Figure 14 cross-sectional and frontal view of yet an exemplary orthokeratology rigid contact lens embodiment, not to scale, as disclosed herein.

DETAILED DESCRIPTION

The detailed discussion on the prior art, and the subject matter of interest in general, is provided here as the background of the present disclosure, to illustrate the context of the disclosed embodiments, and furthermore, to distinguish the advances contemplated by the present disclosure over the prior art.

No material presented here should be taken as an acknowledgment that the material mentioned is previously disclosed, known, or part of common general knowledge, on the priority of the various embodiments and/or claims set forth in the present disclosure.

The following description is provided in relation to several embodiments that may share common characteristics and features of the disclosure. It is to be understood that one or more features of one embodiment may be combined with one or more features of any other embodiments to constitute additional embodiments.

The present disclosure will be described in detail with reference to one or more embodiments, some of which are illustrated and supported by accompanying figures. The examples and embodiments are provided by way of explanation and are not to be construed as limiting to the scope of this disclosure.

The terms "comprise", "comprises", "comprising", "having", "including", "includes" or any variation thereof, are intended to reference a non-exclusive inclusion, such that a device, a method, a process or an apparatus that comprises a list of individual elements does not include only those elements recited, but may also include other elements not expressly listed and equivalents inherently known or obvious to those of reasonable personal of skill in the art.

Other combinations of structures, arrangements, applications, proportions, elements, materials, or components used in the practice of the invention, in addition to those not specifically recited, may be varied, or otherwise particularly adapted to specific environments, manufacturing specifications, design parameters or other operating requirements without departing from the scope of the invention and are intended to be included in this disclosure.

Throughout this disclosure, various exemplary embodiments, known to the inventors at this time, of the invention are disclosed. These embodiments and modes are not intended to limit the scope, applicability, or configuration or the invention in any way. Rather, the following disclosure is intended to teach both the implementation of the exemplary embodiments, modes and any equivalent modes or embodiments that are known or obvious to those of reasonable skill in the art. Additionally, all included figures are non-limiting illustrations of the exemplary embodiments and modes, which similarly avail themselves to any equivalent modes or embodiments that are known or obvious to those of reasonable skill in the art. In this section, the present disclosure will be described in detail with reference to one or more embodiments, some are illustrated and supported by accompanying figures. The examples and embodiments are provided by way of explanation and are not to be construed as limiting to the scope of the disclosure. The functional and structural information disclosed herein is not to be interpreted as limiting in any way and should be construed merely as a representative basis for teaching a person skilled in the art to employ the disclosed embodiments and variations of those embodiments in various ways. The sub-titles and relevant subject headings used in the detailed description section have been included only for the ease of reference of the reader and in no way should be used to limit the subject matter found throughout the invention or the claims of the disclosure. Further, the sub-titles and relevant subject headings should not be used in construing the scope of the claims or the claim limitations.

The human visual system is continuously challenged with the categorisation of intricate visual stimuli that involve both natural and man-made scenes. The characteristics of these visual scenes can be either understood using spatial domain analysis, for example, by describing a scene in terms of pixel luminance, brightness, intensity, or colour as function of pixel spacing. On the contrary, a scene can also be analysed using a Fourier domain analysis, which essentially involves decomposing a visual scene into dual representations created using the amplitude and phase spectra of the visual scene. The amplitude spectrum corresponds to the distribution of luminance contrast across spatial frequencies and orientations, and the phase spectrum corresponds to the spatial relation between spatial frequencies within the visual scene. The magnitude of luminance variation in a visual scene relative to its mean luminance is referred to as luminance contrast.

The visual system utilises low-level features like spatial frequencies and luminance contrast to enable recognition, and from a neurobiological point of view, it is known that cells both at the retinal and visual cortex level respond to luminance contrast, spatial frequencies, and orientations. The coarse information contained in lower spatial frequencies within an image are capable of conveying plausible interpretations of the visual scene. This initial coarse information further guides processing of high spatial frequencies to offer finer information of the visual scene at the visual cortex of an individual.

A Fourier analysis of a visual scene captures a large amount of image redundancy since the power spectrum of a visual scene (i.e., the square of its amplitude spectrum) provides a direct measure of its autocorrelation. Some parts of this current disclosure focuses on a Fourier analyses of the visual scenes both natural and man-made scenes. Particularly, the published paper by Torralba and Oliva titled "Statistics of natural image categories", published in Computational Neural Systems, volume 14 (2003), pages: 391-412. The mean power spectrum can be modelled using polar coordinates in which the shape of the spectra is predominantly a function of the orientation characteristics within the visual scene or image.

Throughout the disclosure, this may be referred to as a spectral signature of a visual scene or image, with a particular focus on the characteristics of the spectral signature discussed herein. The characteristics of the spectral signature relevant to the disclosure include the shape of the spectral signature and the energy distribution in the horizontal and vertical orientation when compared with the energy distribution in the oblique orientations. In real-world visual scenes, including both the natural and man-made ones, the vertical and horizontal orientations are much more frequent than oblique orientations. In other words, the spectral signature of the real-world visual scenes, both the natural and man-made ones, have more energy distributed in the horizontal and vertical orientations than in the oblique orientations. Upon a closer inspection of the oblique orientations within the spectral signatures of natural and man-made visual scenes, it can be noted that the oblique orientations are more frequent in natural scenes than man-made scenes. Further, in some man-made scenes, the oblique orientations may almost be non-existent or negligibly small.

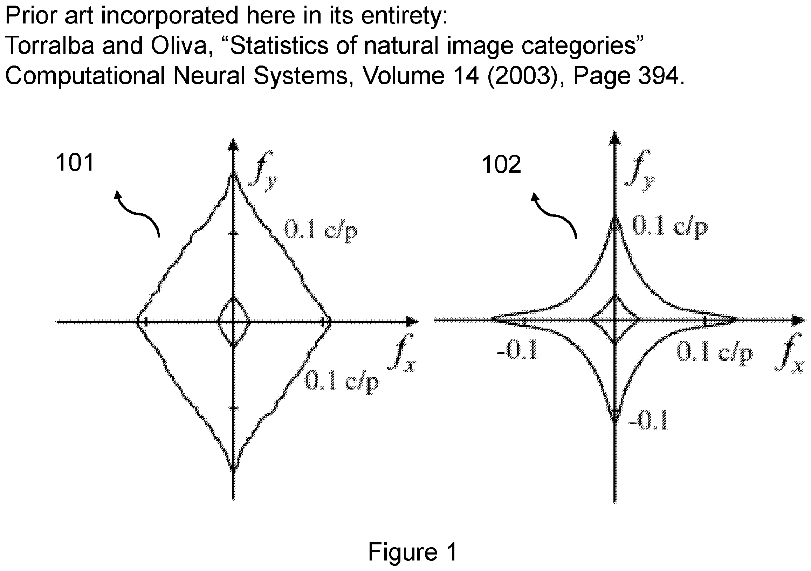

Figure 1 is reproduced from a prior art publication, [Torralba et al, "Statistics of natural image categories", Computational Neural Systems, 14 (2003), page: 394]. Figure 1 highlights the spectral signatures of two distinct types of visual scenes, a representative sample (101) obtained by averaging numerous images of natural scenes and another sample (102) obtained by averaging numerous images of man-made scenes. Post-processing of a number of representative natural and man-made images results in the depicted spectral signatures (101 and 102). The two contour lines within the spectral signatures represent the 50% (inner) and 80% (outer) of the energy captured in the spectral signature. The markings on the x- and y-axis of 101 and 102 represent an arbitrary spatial frequency threshold of 0.1 cycles per pixel. The spatial frequency scales used in this example are normalised. The distinct difference between 101 and 102 can be attributed to the isotropy in the spectral signature or lack thereof (i.e., anisotropy). The representative natural image provides a more isotropic spectral signature with energy component observed in horizontal, vertical, and oblique directions. On the contrary, man-made representative scenes provides anisotropic spectral signatures with minimal amount of energy captured in the oblique direction. On a much smaller scale of differences, more relative energy is captured in the horizontal dimension of the spectral signature, when considering a man-made scene. On the other hand, more relative energy is captured in the vertical dimension of the spectral signature, when considering a natural scene.

This disclosure contemplates that the anisotropic distribution of orientations in the images may have the ability to tune the neurobiology of a pre-myopic or a myopic eye, which is otherwise predominantly exposed to one type of image or scene over another type. For example, myopic eyes which are exposed to more man-made scenes may develop appropriate neurobiological changes to adapt to the corresponding visual impetus.

In other words, this disclosure contemplates that man-made scenes or lack of oblique frequencies in a scene create a stimulus for eye growth. On the contrary, when the eyes are exposed to natural images, or images that offer oblique frequencies, the images may offer an optical stop signal that can decelerate eye growth or progression of myopia.

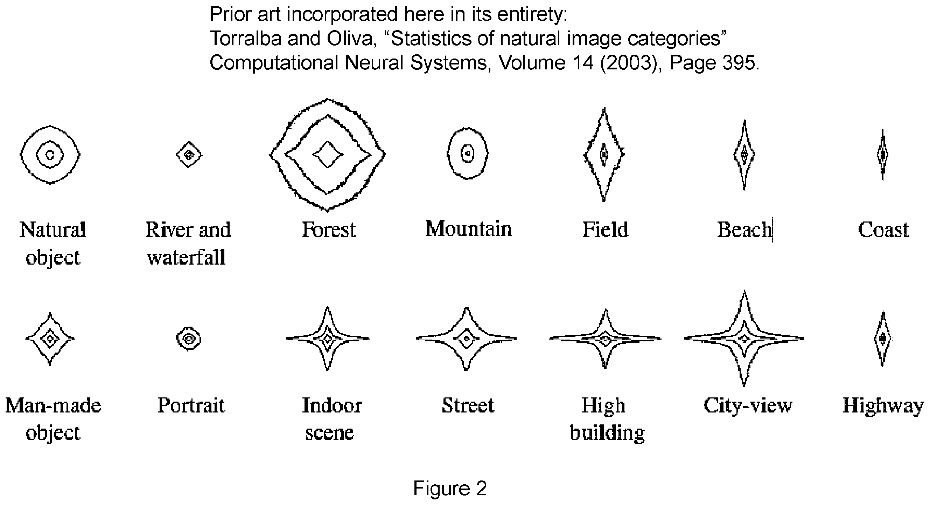

Figure 2 is also reproduced from a publication of prior art, [Torralba et al, "Statistics of natural image categories", Computational Neural Systems, 14 (2003), page: 395]. Figure 2 showcases the spectral signatures of about 14 different types of scenes, which include 7 representations of scenes that are of a natural origin and another 7 man-made scenes. Post-processing (for example, averaging the power spectra) of the 14 representative images in each category results in the depicted spectral signatures. Figure 2 further highlights the distinct contour plots of the 14 spectral signatures, wherein the three contour lines within each spectral signature represents the 60%, 80% and 90% of the energy captured. The sum of the squared Fourier components results in the contour plots depicting the total energy spread. Although the representative scenes are depicting distance visual scenes, the concepts may be extrapolated to spectral signatures of intermediate or near visual scenes; for example, indoor settings viewing a computer, laptop, tablet, book, smart phone, etc.

The averaged power spectra from the 14 different categories of scenes exhibit distinctly different orientations and spatial frequency distributions as noticed herein. The differentiation amongst various man-made categories predominantly resides in the relationship between horizontal and vertical contours at different scales with very minimal energy being spread in the oblique directions of the spectral signature. However, the spectral signatures of natural environments seem to have a much broader variation in spectral shapes. The spectral signature of a few individual scene categories is strikingly different when considering their basic class level, for example a forest scene versus a city scene. From the contour plots of the Figure 2, as noticed herein, the dominant spatial scales and dominant orientations are very typical of classes of scenes representing different volumes or depth ranges. The spectral signatures of images of large-scale scenes are dominated by the horizontal orientation, for example, a beach scene versus a coast scene. When the scene background becomes closer to the observer, for example considering an image of a mountain compared to an image of an enclosed natural object, the spectral signatures become more isotropic and appears denser in high spatial frequencies. The shape of the spectral signatures in natural environment seems to be correlated with the scale or size of the main components of the image, for example, finer texture observed in a forest scene versus a much coarser texture observed in waterfalls.

This current disclosure postulates that the differences between natural and man-made imagery may be a plausible explanation for progressive myopia. Further, this disclosure contemplates that the anisotropic energy distribution within the spectral signature of the image formed on the retina, or spectral signatures lacking oblique orientations, may have the ability to tune the neurobiology of a pre-myopic or a myopic eye which is predominantly exposed to one type of an image or scene over another type. For example, myopic eyes which are exposed to more man-made scenes than natural scenes may develop appropriate neurobiological changes to adapt the corresponding visual impetus. For instance, a population-based evidence or observation supporting the proposed hypothesis is that children who live or grow-up in the rural, villages, smaller towns, or country-side are less likely to develop myopia than their counterparts who live or grow-up in the urban or modern-day cities.

The present invention proposes an alternative method to retard the progression of myopia by purposefully introducing or increasing the energy spread into the oblique dimensions of the retinal spectral signature by utilisation of non-circular non-transparent aperture stop on an otherwise single vision contact lens as disclosed herein. In some examples, the purposeful introduction or increase of the energy spread into the oblique dimensions of the retinal spectral signature may be limited to a specific region on the retina or may not be limited to any specific region on the retina. In some embodiments, the contact lenses may include soft contact lenses, while in yet another embodiment, the contact lens may mean rigid or hard contact lenses, worn either during the day or at night for orthokeratology purpose (flattening the shape of the anterior surface of the eye, cornea). Another embodiment the disclosure includes an alternative method to retard the progression of myopia by purposefully increasing the energy spread in the oblique dimensions of the retinal spectral signature by utilising non-circular flattened zones on the cornea using specifically designed orthokeratology lenses with dedicated back surface design discussed herein such that the orthokeratology lenses are configured to provide a non-circular treatment zone. In such examples, it is understood that the non-circular treatment zone of the flattened cornea may produce a quasi-aperture. In other words, in such an instance the aperture is not formed by opaque boundaries but rather a change corneal refractive power in a non-circular function, also referred as quasi-aperture herein.

For example, eyes which are exposed to more man-made scenes devoid of oblique frequencies, whether at distance and/or near visual setting, may develop appropriate neurobiological changes to adapt to such corresponding visual impetus and further accelerate myopia. In other words, this invention disclosure contemplates that man-made scenes or lack of oblique frequencies in a scene create a stimulus for further eye growth. On the contrary, when eyes are exposed to images that have energy spread into oblique frequencies may experience a stop signal to eye growth.

The cone and rod receptors in the central and peripheral retina are arranged differently in terms of both size and spacing. In some embodiments of the disclosure, the power spectrum analysis of a visual scene may also take these factors into account. Furthermore, there may be interactions between the power spectrum of a visual scene and the wavelength spectrum of a visual scene.

The wavelength spectrum of a visual scene includes the wavelength characteristics of the incoming light. In some visual scenes, the wavelengths of a visual scene may be predominately biased towards blue end of the visual spectrum, approximately 420 nm and 490 nm, inclusive. In some other visual scenes, the wavelengths of a visual scene may be predominately biased towards green band of the visual spectrum, approximately 500 nm and 590 nm, inclusive.

In yet some other visual scenes, the wavelengths of a visual scene may be predominately biased towards red end of the visual spectrum, approximately 600 nm and 760 nm. In yet some other visual scenes, the wavelengths of a visual scene may be predominately biased towards human cone sensitivity function, which is a nonlinear function with a peak sensitivity towards approximately 555 nm.

A contact lens device or method providing a stop-signal to retard the rate of eye growth, or stop the eye growth, or the state of refractive error of the wearer's eye, based on employing a non-circular non-transparent aperture stop within an otherwise single vision optic zone may facilitate an altered point spread function and modulation transfer function describing an energy distribution on the retina; wherein the energy distribution on the retina of the said altered point spread function is formed such that the energy is distributed into the oblique frequencies, which may serve as an optical stop signal for inhibiting, reducing, or controlling progressive myopic refractive error.

The present invention relates to a device, and/or method for reducing the rate of myopia progression, and particularly to an advanced contact lens configured with a non-circular non-transparent aperture stop employed within the optic zone of the lens designed to purposefully introduce energy into oblique frequencies of the retinal spectral signature, wherein the altered retinal imagery may provide a stop signal for the growth of a myopic eye.

The present disclosure also relates to an orthokeratology contact lens configured to result in a non-circular effective quasi-aperture, to correct the distance refractive error of the wearer. Wherein, the effective non-circular quasi-aperture alters the transmitted properties of the incoming light, artificially introducing oblique frequencies that are otherwise observed only in natural viewing scene. These oblique frequencies may produce a stop signal for reducing myopia progression.

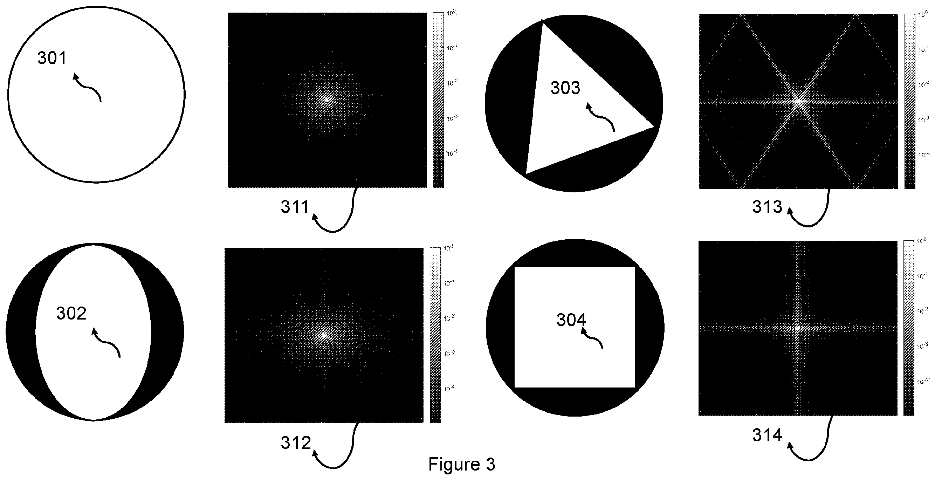

Figure 3 shows the retinal point spread function obtained when collimated light passes through a circular (301), an elliptical (302), a triangular (303) and a square (304) apertures of a schematic model eye. The on-axis point spread transfer function of the schematic model eyes configured with a circular aperture stop or pupil is showcased in 311. As can be noticed herein (311), the energy is distributed rotationally symmetrically across all the meridians, including the horizontal, vertical, and oblique directions. Unlike the retinal point spread function observed with use of a circular aperture (301), the retinal point spread function obtained with use of an elliptical aperture stop or pupil is rotationally asymmetric, particularly observing the variations seen between the horizontal and vertical meridians. Furthermore, the use of a triangular aperture stop or pupil within the schematic eye, provides a redistribution of energy into oblique frequencies of its retinal point spread function (313). On the other hand, the retinal point spread function (314) observed with use of rectangular aperture (304), completely mitigates, or weakens the oblique frequencies.

As contemplated by this disclosure, the triangular aperture (303) and its corresponding retinal point spread function (313) would be preferred to be configured in the devices disclosed herein and not the rectangular aperture (304) as its corresponding retinal point spread function (314), is devoid of oblique frequencies that are observed in the images casted from a natural scene.

In some other embodiments of the disclosure, the orientation of the polygonal pupil may be configured to selectively mitigate or weaken specific oblique frequencies over other oblique, horizontal, or vertical frequencies.

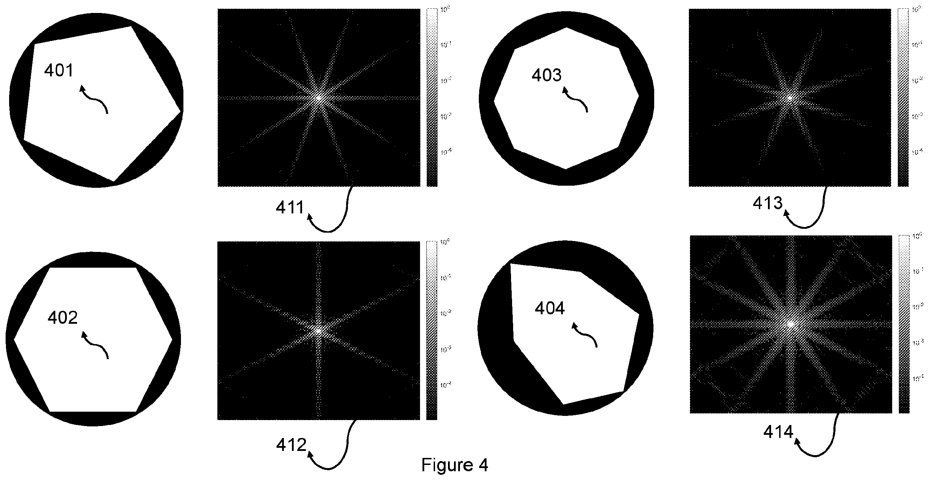

Figure 4 shows the retinal point spread function observed when collimated light passes through a pentagonal (401), hexagonal (402), octagonal (403) and irregular hexagonal (404) apertures of a schematic model eye. The on-axis point spread function of the schematic model eye configured with a pentagonal, hexagonal, octagonal, and irregular hexagon aperture stop or pupils are showcased in 411, 412, 413 and 414 respectively. As contemplated by this disclosure, the pentagonal (401), hexagonal (402), octagonal (403) and irregular hexagon shaped (404) apertures and its corresponding point spread function (411, 412, 413 and 414 respectively) would be preferred aperture types to be configured within the contemplated optical devices (i.e., contact lenses) disclosed herein. In this example, the use of such apertures would redistribute the energy to the oblique frequencies, potentially mimicking the optical performance obtained while viewing natural images, or viewing visual scenes casted from a natural scene. In some other embodiments, the orientation of the polygonal pupil may be configured to purposefully mitigated or weakened in certain desired oblique frequencies over other oblique, horizontal, or vertical spatial frequencies.

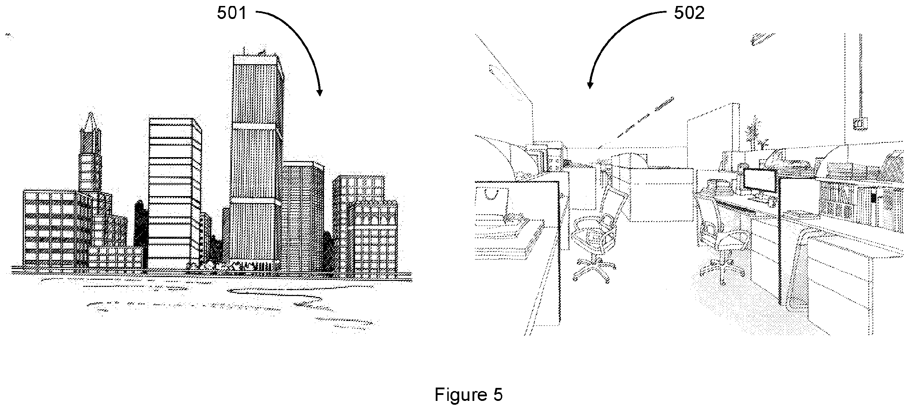

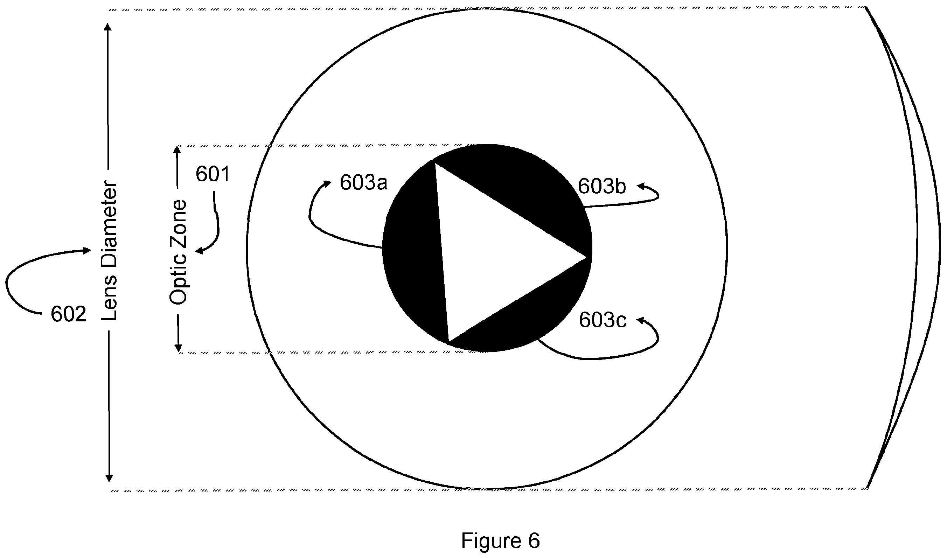

Figure 5 showcases sketches of two man-made scenes that are utilised for the evaluation of the performance of spectral signatures of the retinal images formed by one or more embodiments. The first man-made scene is a sketch of a representation of a city-skyline and the second man-made scene is a representation of an indoor office setting. To facilitate the evaluation, the two man-made scenes of Figure 5 were considered as source bitmap files, wherein these source bitmap files were convolved with an array of point spread functions computed at the retina of the schematic model eye, the array of point spread functions spanning a range of field angles on the retina. The performance was obtained when one or more embodiments of the disclosure were used to correct an appropriate schematic myopic model eye, described in the embodiment examples 1 to 5 (Figures 6 to Figure 13) herein. The grid sampling for computing the point spread functions was set to 65536 (256 x 256 array). The performance evaluation depicted in embodiment Examples 1 to 4 were performed at different configurations, including different pupils, wavelengths, and field angles.

The computation of the spectral signatures for each of the embodiment Examples 1 to 4 consists of at least the following steps: (i) a source bitmap, for example, one of the two man-made images of Figure 5, is oversampled by at least a factor of 2; (ii) at least determine the number of point spread functions to be computed in each direction over the desired field of view, wherein the number of point spread functions in each direction over the desired field of view is at least 11 x 11 array; (iii) third, compute the at least 11 x 11 array grid of point spread functions over the desired field angle on the retina of the schematic model eye fitted with various embodiments of the present disclosure; wherein the array grid of point spread functions are computed using Huygens' diffraction principles that takes into account, both aberrations of the system and diffraction effects around the edges of the shape of the arbitrary aperture stop of the current disclosure; (iv) each point spread function within the array grid of point spread functions is then interpolated for every pixel in the modified source bitmap; wherein at each pixel, the effective point spread function is convolved with the modified source bitmap to determine the resulting aberrated bitmap image; wherein the resulting image bitmap I(x, y) is finally scaled and stretched to account for the desired image pixel size, distortion, and lateral aberrations, if any; (v) perform a Fourier domain analysis of the resulting bitmap image I(x, y) and deduce the normalised power spectrum resulting in the spectral signature using the formula in Equations 1-2; and (vi) finally, the data within the normalised power spectrum obtained through Equations 1-2 may be rescaled, such that at least 60%, or 70%, or 80% of the energy is captured within the power spectrum may be retained.

$$I_{fft}(x, y) = abs\left(fftshift(fft2(I(x, y)))\right) - \text{Equation 1}$$

$$Normalised\ power\ spectrum = \log\left(\frac{I_{fft}(x, y)}{max\left(\max\left(\left(I_{fft}(x, y)\right)\right)\right)}\right) - \text{Equation 2}$$

Schematic model eyes were used for computation of the spectral signatures of the exemplary embodiments of the current disclosure. The prescription parameters of the schematic model eyes used for the computation of the spectral signatures are tabulated in Table 1. The prescription in Table 1 should not be construed as an imperative method to demonstrate the effect with the contemplated exemplary embodiments of the disclosure.

It is just one of many methods that may be used by the person skilled in the art for optical simulation purposes. In other examples, a lens designer may also alter the parameters of the individual parameters of the model eye; for example, the cornea, lens, retina, media, or combinations thereof, to aid a better simulation of the effect being described. For example, to demonstrate the effects of other embodiments, other schematic model eyes like Atchison, Escudero-Navarro, Liou-Brennan, Polans, Goncharov-Dainty may also be used instead. In other examples, a physical or a bench-top model eye may be used in place of a schematic model eye.

Table 1: Prescription of a schematic myopic model eye with prescription of -3 DS.

| Type | Comment | Radius (mm) | Thickness (mm) | Refractive Index | Semi Diameter (mm) | Conic Constant |
|------|---------|-------------|----------------|------------------|--------------------|----------------|
| **Standard** | | Infinity | Infinity | | 0 | 0 |
| **Standard** | Start | Infinity | 5 | | 4 | 0 |
| **Standard** | Anterior Cornea | 7.75 | 0.55 | 1.376 | 5.75 | -0.25 |
| **Standard** | Posterior Cornea | 6.4 | 3 | 1.334 | 5.5 | -0.4 |
| **Standard** | Pupil | Infinity | 0.45 | 1.334 | 5 | 0 |
| **Standard** | Anterior Lens | 10.8 | 3.8 | 1.423 | 4.5 | -4.798 |
| **Standard** | Posterior Lens | -6.25 | 17.72 | 1.334 | 4.5 | -4.101 |
| **Standard** | Retina | -12 | 0 | | 6 | 0 |

The schematic model eye of Table 1 is corrected with a conventional or traditional single vision contact lens; however, the optic zone of the single vision lens was approximately 6 mm diameter, smaller than a conventional single vision contact lens. The following parameters were used to emulate the single vision contact lens; front surface radius of 8.675 mm and asphericity Q of zero and the posterior surface radius of 8.13 with an asphericity Q of -0.13. To emulate one or more of the embodiments of the present disclosure, the transmission properties of the optic zone were altered as indicated in embodiment Examples 1 to 4 described herein.

Exemplary Example 1

Figure 6 shows the frontal view an exemplary contact lens embodiment, not to scale. The frontal view illustrates an optic zone (601), a lens diameter (602) and the design features (603a to 603c) i.e., non-circular non-transparent features of an aperture stop. In this example, the lens diameter is approximately 14 mm in diameter, the optic zone is designed substantially with single vision refractive power and is approximately 6 mm in diameter.

In this example, the featured non-circular non-transparent region is the omitted region within the circle of diameter 6 mm inscribed with an equilateral triangle, whose three sides have a defined length of 2*R*cos(30 degrees), wherein R = 3 mm. In this example, the area of the transparent region within the optic zone, defined by the equilateral triangle, is approximately 11.66 square millimetres. In this example, the area of the non-circular non-transparent region, is approximately 16.6 square millimetres.

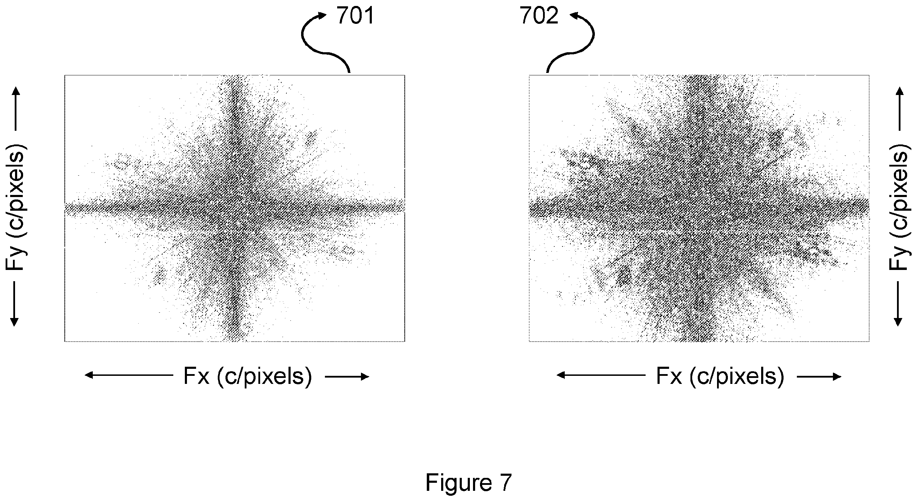

Figure 7 shows the spectral signatures of the retinal images formed by one of the exemplary embodiments disclosed in Figure 6 fitted on a schematic myopic model eye described in Table 1. The single vision contact lens described in paragraph [0088] is configured in two different configurations, (a) without any contemplated non-circular non-transparent features, and (b) with the contemplated non-circular non-transparent aperture stop features described in Figure 6 (603). When compared with the spectral signature of the retinal image (701) obtained with single vision contact lenses without any features of the disclosure, the use of non-circular non-transparent aperture stop (603) configured within an otherwise single vision contact lens facilitated the redistribution of the light energy into the oblique frequencies of the spectral signature of the retinal image (702), which may serve as a trigger for a stop signal to a progressing myopic eye. In this example, the spectral signature represents 60% of the energy captured in the power spectrum analysis (701 & 702) described herein. In other examples, additional power spectrum analysis to include at least 70% or 80% of the captured energy within the spectral signature may be desired. In this example, the computation was done using the following parameters: (a) 5 mm pupil diameter on the model eye; (b) a monochromatic wavelength of 0.589 microns; (c) a 15-degree field angle and (d) indoor man-made scene (501) described in Figure 5.

Exemplary Example 2

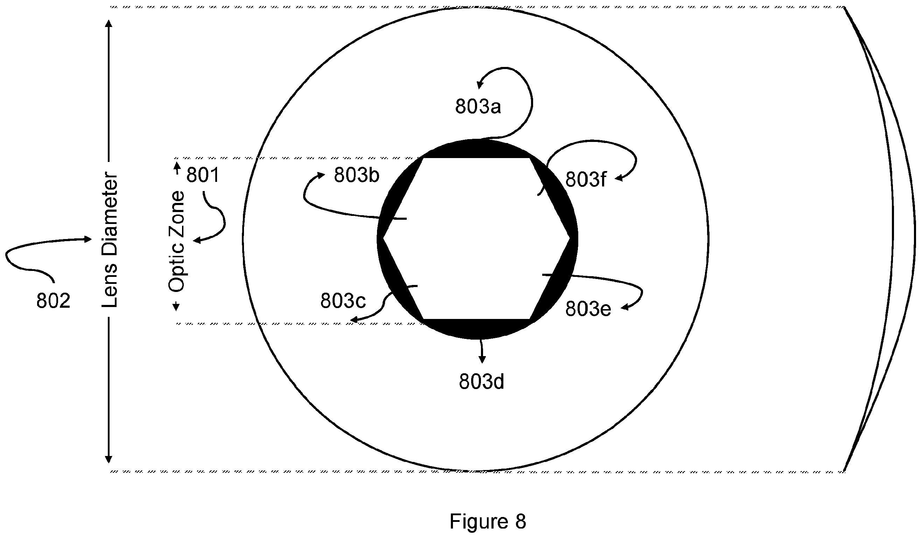

Figure 8 shows the frontal view an exemplary contact lens embodiment, not to scale. The frontal view illustrates an optic zone (801), a lens diameter (802) and the design features (803a to 803f) i.e., non-circular non-transparent features of an aperture stop. In this example, the lens diameter is approximately 14.2 mm in diameter, the optic zone is designed substantially with single vision refractive power and is approximately 6 mm in diameter.

In this example, the featured non-circular non-transparent region is the omitted region within the circle of 6 mm inscribed with a regular hexagon, whose six sides have a defined length of R, wherein R = 3 mm. In this example, the area of the transparent region within the optic zone, defined by the regular hexagon, is approximately 23.38 square millimetres. In this example, the area of the non-circular non-transparent region, is approximately 4.88 square millimetres.

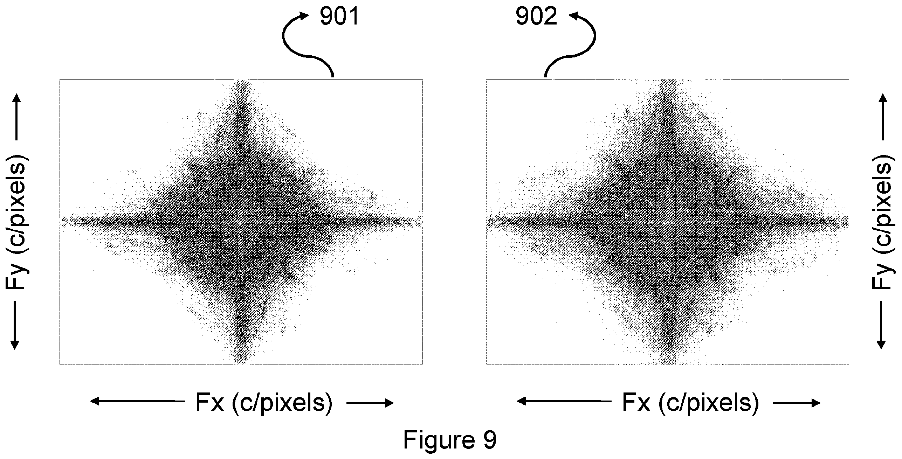

Figure 9 shows the spectral signatures of the retinal images formed by one of the exemplary embodiments disclosed in Figure 8, fitted on a schematic myopic model eye described in Table 1. The single vision contact lens described in paragraph [0088] is configured in two different configurations, (a) without any contemplated non-circular non-transparent features, and (b) with the contemplated non-circular non-transparent aperture stop features described in Figure 8 (803). When compared with the spectral signature of the retinal image (901) obtained with single vision contact lenses without any features of the disclosure, the use of non-circular non-transparent aperture stop (803) configured within an otherwise single vision contact lens facilitated the redistribution of the light energy into the oblique frequencies of the spectral signature of the retinal image (902), which may serve as a trigger for a stop signal to a progressing myopic eye. In this example, the spectral signature represents 70% of the energy captured in the power spectrum analysis (901 & 902) described herein. In other examples, additional power spectrum analysis to include at least 50% or 80% of the captured energy within the spectral signature may be desired. In this example, the computation was done using the following parameters: (a) a 5 mm pupil diameter on the model eye; (b) a polychromatic wavelength source representing photopic luminosity function; (c) a 20-degree field angle and (d) an indoor man-made scene (501) described in Figure 5.

In this example, using a transparent region using regular hexagonal aperture may result in a non-circular non-transparent region that is too narrow to be effective in all conditions. Wherein, either too much light is blocked, or the natural pupil shapes the aperture overrides the features of the contemplated disclosure. In such instances, the design features may be optimised to deal with the issue of variable pupil size due to variable illumination conditions that a wearer may be experiencing in a daily situation. For example, one such optimisation of the design feature may include separating the six lines of the hexagonal aperture (803a to 803f) and configured to be arranged at different distance from the centre. Wherein, at least one of the edges will influence the power spectrum, while maintaining some regulation of the light flux. Alternatively, in some other embodiment, the optimisation of such a design feature may include decentration of the proposed hexagonal (803) aperture.

Exemplary Example 3

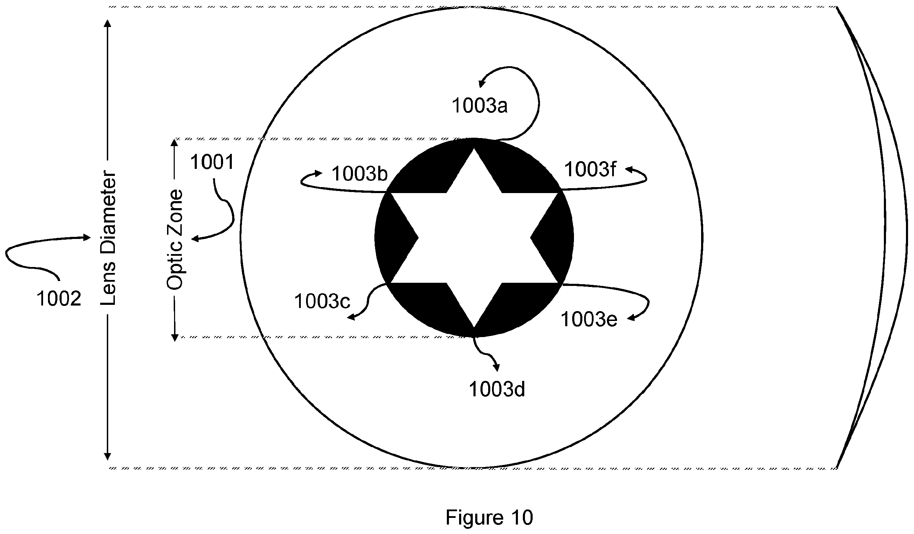

Figure 10 shows the frontal view an exemplary contact lens embodiment, not to scale. The frontal view illustrates an optic zone (1001), a lens diameter (1002) and the design features (1003a to 1003f) i.e., non-circular non-transparent features of an aperture stop. In this example, the lens diameter is approximately 13.8 mm in diameter, the optic zone is designed substantially with single vision refractive power and is approximately 6 mm in diameter.

In this example, the featured non-circular non-transparent region is the omitted region within the circle of diameter 6 mm inscribed with a transparent region defined as a regular shaped star. In this example, the area of the transparent region within the optic zone, defined by the regular shape star, is approximately 23.32 square millimetres. In this example, the area of the non-circular non-transparent region, is approximately 4.94 square millimetres.

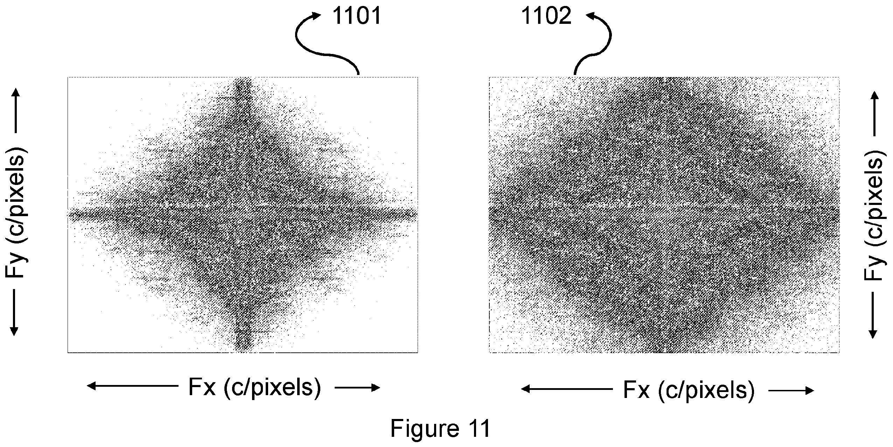

Figure 11 shows the spectral signatures of the retinal images formed by one of the exemplary embodiments disclosed in Figure 10, fitted on a schematic myopic model eye described in Table 1. The single vision contact lens described in paragraph [0088] is configured in two different configurations, (a) without any contemplated non-circular non-transparent features, and (b) with the contemplated non-circular non-transparent aperture stop features described in Figure 10 (1003).

When compared with the spectral signature of the retinal image (1101) obtained with single vision contact lenses without any features of the disclosure, the use of non-circular non-transparent aperture stop (1003) configured within an otherwise single vision contact lens facilitated the redistribution of the light energy into the oblique frequencies of the spectral signature of the retinal image (1102), which may serve as a trigger for a stop signal to a progressing myopic eye.

In this example, the spectral signature represents 60% of the energy captured in the power spectrum analysis (1101 & 1102) described herein. In other examples, additional power spectrum analysis to include at least 80% or 90% of the captured energy within the spectral signature may be desired.

In this example, using a transparent region using regular shape star apertures may result in a non-circular non-transparent region that may be too narrow to be effective in all conditions. Wherein, either too much light is blocked, or the natural pupil shapes the aperture overrides the features of the contemplated disclosure both of which are not desirable situations. In such instances, the non-circular non-transparent design features may be further optimised to deal with the issue of variable pupil size due to variable illumination conditions that a wearer may be experiencing in a daily situation. For example, one such optimisation of the design feature may include separating the six smaller triangles of the regular star shaped aperture (1003a to 1003f) and be reconfigured to be arranged at different distance from the centre. Wherein, at least one of the edges will influence the power spectrum of the spectral signature of the retinal image, while maintaining some desirable regulation of the light flux. Alternatively, in some other embodiments of the disclosure, the optimisation of such a design feature may also include decentration of the proposed regular shaped star aperture to have an irregular feature. The use of modified non-circular non-transparent aperture stop features (1003) configured within a contact lens is set to distribute the energy into the oblique frequencies of the spectral signature of the on- and/or off-axis retinal image may be contemplated to trigger a stop signal to progressing myopic eye. Various other physiological considerations of the individual myopic eye may also be factored into the optimisation of design features such that there is an overall balance achieved between the visual performance with the contact lens while maintaining the stimulus to slow the progression of myopia.

In this example, the computation was done using the following parameters: (a) a 6 mm pupil diameter on the model eye; (b) a polychromatic wavelength source representing photopic luminosity function; (c) a 20-degree field angle and (d) an outdoor man-made scene (502) described in Figure 5.

Exemplary Example 4

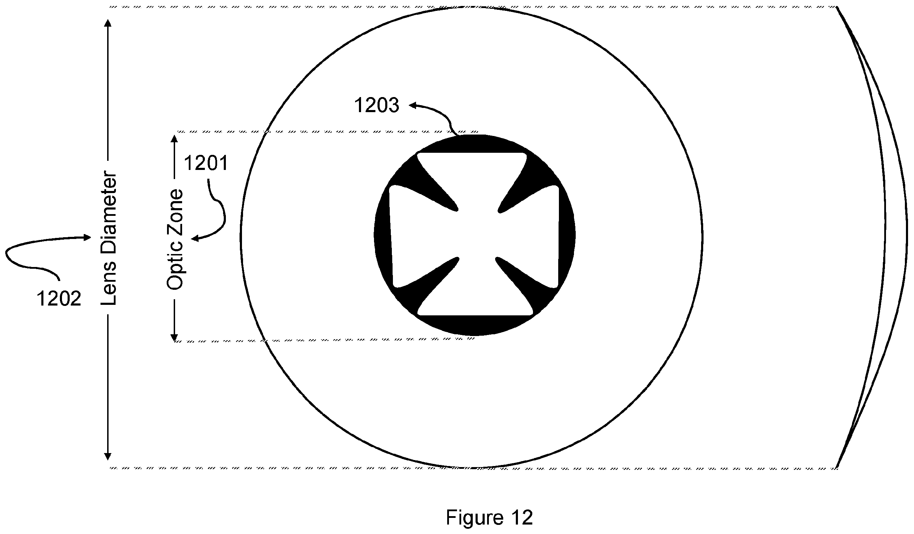

Figure 12 shows the frontal view an exemplary contact lens embodiment, not to scale. The frontal view illustrates an optic zone (1201), a lens diameter (1202) and the design features (1203) i.e., non-circular non-transparent features of the aperture stop. In this example, the lens diameter is approximately 14 mm in diameter, the optic zone is designed substantially with single vision refractive power and is approximately 6 mm in diameter.

In this example, the featured non-circular non-transparent region is the omitted region within the circle of diameter 6 mm inscribed with a regular shape Maltese cross. In this example, the area of the transparent region within the optic zone, defined by the regular shape, is approximately 24 square millimetres. In this example, the area of the non-circular non-transparent region, is approximately 4 square millimetres.

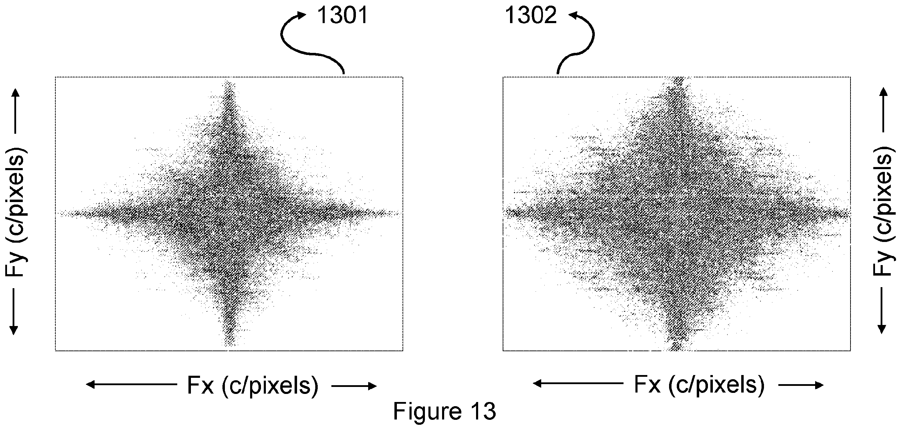

Figure 13 shows the spectral signatures of the retinal images formed by one of the exemplary embodiments disclosed in Figure 12, fitted on a schematic myopic model eye described in Table 1. The single vision contact lens described in paragraph [0088] is configured in two different configurations, (a) without any contemplated non-circular non-transparent features, and (b) with the contemplated non-circular non-transparent aperture stop features described in Figure 12 (1203).

When compared with the spectral signature of the retinal image (1301) obtained with single vision contact lenses without any features of the disclosure, the use of non-circular non-transparent aperture stop (1203) configured within an otherwise single vision contact lens facilitated the redistribution of the light energy into the oblique frequencies of the spectral signature of the retinal image (1302), which may serve as a trigger for a stop signal to a progressing myopic eye.

In this example, the spectral signature represents 60% of the energy captured in the power spectrum analysis (1301 & 1302) described herein. In other examples, additional power spectrum analysis to include at least 80% or 90% of the captured energy within the spectral signature may be desired.

In this example, the computation was done using the following parameters: (a) a 6 mm pupil diameter on the model eye; (b) a monochromatic wavelength source of 0.589 microns; (c) a 25-degree field angle and (d) an outdoor man-made scene (502) described in Figure 5.

In another contact lens embodiment, the optic zone may have astigmatic optics to correct the distance refractive error of the wearer. In yet another contact lens embodiment, the optic zone may have aspheric optics, i.e., introduction of spherical aberration (positive or negative). In yet another contact lens embodiment, the non-circular non-transparent aperture may be elliptical, triangular, square, rectangular, pentagonal, hexagonal, heptagonal, octagonal, star shaped or another type of polygon shape.

In yet another contact lens embodiment, the designed feature i.e., non-circular non-transparent aperture stop may be contained within the central 1, 2, 3, 4, 5 or 6 mm of the optic zone of the contact lens. In yet another contact lens embodiment, the design feature i.e., non-circular non-transparent aperture stop may be contained between the central 1 mm and 3 mm, or the central 2 mm to 4 mm, or the central 3 mm to 5 mm or the central 2 mm to 6 mm of the optic zone of the contact lens. The choice of the size of the non-circular non-transparent aperture stop may taken into consideration the maximum and minimum variation of the physiological sizes of the pupil. In certain other contact lens embodiments, the contemplated design features of the contact lens may be located, formed, or placed on one of the two surfaces of the contact lens and the other surface may have additional features for further reducing eye growth. For example, use of additional features like defocus, astigmatism, or spherical aberration. To avoid the situation of lower than adequate transmittance into the wearer's eye, the size of the non-circular non-transparent aperture is selected so that the overall transmittance through the contemplated ophthalmic lens is at least 85%, 90%, or 95% of a standard single vision lens of prior art.

In some embodiments of the present disclosure, the stop signal may be configured using non-circular non-transparent aperture stop, that is otherwise designed with single vision optics without significant spherical aberration. In yet some other embodiments, the optic zone of a soft or rigid contact lens may be otherwise be designed with single vision optics that has either positive or negative spherical aberration. In some other embodiments, the optic zone of a soft or rigid contact lens may otherwise be designed with single vision optics that includes both sphere and cylinder power to correct the distance refractive error of the wearer.

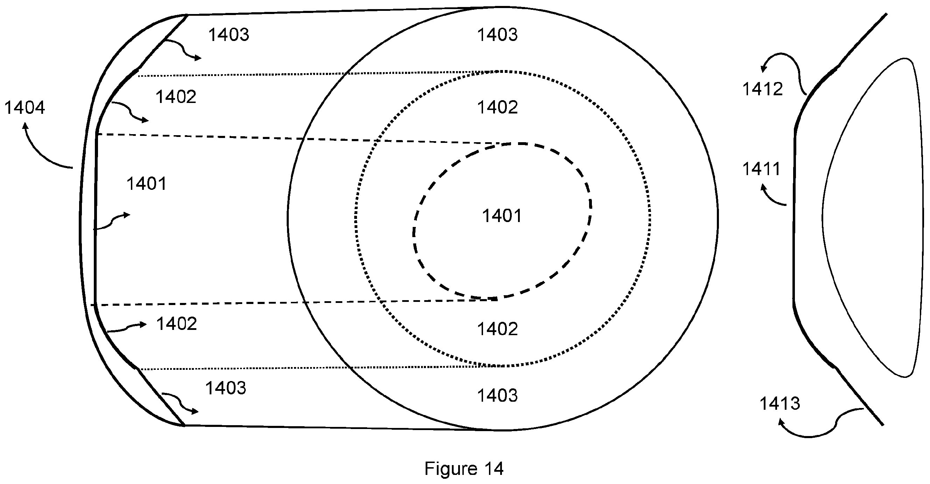

Figure 14 illustrates a rigid gas permeable orthokeratology contact lens embodying the principles of the present disclosure. The contemplated lens of the disclosure when positioned over the cornea of a myopic eye and is configured to have ocular bearing areas on the central cornea (1411), and other selected areas of the cornea (1412, 1413) of the myopic eye, so that when the eyelid (not shown in the figure) is closed, confirms uniform pressure over the lens allowing reshaping of the cornea. A selective arrangement of the curvatures of the posterior surface of the lens (1401, 1402 and 1403) based on the topographical characteristics of the corneal surface of the myopic eye (1411, 1412, and 1413) defines the ocular bearing regions, allowing the reshaping of the corresponding corneal portions of the cornea of the myopic eye. According to one of the embodiments of the present disclosure, as illustrated in Figure 14, an orthokeratology rigid gas permeable contact lens for a myopic eye comprising at least three zones, namely, a base-curve zone (1401), an alignment-curve zone (1402) and at least one peripheral-curve zone (1403); the base-curve zone configured over a non-circular region spanning a defined area with a curvature less than a measured curvature of a central corneal region (1411), and creating a primary compressive force to flatten the central corneal region and provide correction for the myopic eye; the alignment-curve zone (1402) surrounding or circumscribing the base-curve zone, configured with a define shape, size, and a curvature greater than the base-curve zone (1401) to create a secondary compressive force to flatten the central corneal region; and the at least one peripheral-curve zone (1403) circumscribing the alignment-curve zone (1402), configured with another define shape, size, and a curvature less than a measured curvature of an underlying mid-peripheral portion of the cornea (1413), to align and centre the lens substantially over the central corneal region; wherein the non-circular base-curve zone (1401) of the lens creates a corresponding non-circular region of flattened cornea (1411), when the lens is applied on the myopic eye, serving as a non-circular quasi-aperture; wherein the light entering the myopic eye through this non-circular quasi-aperture introduces additional at least partial diffractive effects leading to a portion of the light energy being distributed into oblique directions on the retina, observed in as oblique frequencies in the spectral signature, mimicking the visual experience of visualising a natural scene, and thereby producing a stop signal to a progressing myopic eye. In this example, the spectral signature represents 60% of the energy captured in the power spectrum analysis described herein.

The present invention may be used in combination with any of the devices/methods that have the potential to influence the progression of myopia. These may include, but are not limited to, spectacle lenses of various designs, colour filters, pharmaceutical agents, behavioural changes, and environmental conditions that may be considered by a person skilled in the art.

**Claims**

1. A contact lens for a myopic eye, the contact lens comprising an optical zone (601, 801, 1001, 1201) with an optical centre; the optical zone comprising:

   a transparent region with a single vision power profile;
   a non-circular non-transparent region (603, 803, 1003, 1203) circumscribing the transparent region configured to form an aperture stop (603a, 603b, 603c, 803a, 803b, 803c, 803d, 803e, 803f, 1003a, 1003b, 1003c, 1003d, 1003e, 1003f, 1203) of the contact lens;
   wherein the contact lens when **characterised by** testing on a model eye configured with a distance refractive error matching the single vision power profile provides a retinal image (702, 902, 1102, 1302), for at least one pupil diameter between 3 mm and 6 mm inclusive, for at least one wavelength 420 nm to 760 nm inclusive, and for at least a wide field angle at the retina;
   wherein the retinal image (702, 902, 1102, 1302) when further characterised using a power spectrum Fourier transform analysis results in a spectral signature of the retinal image (702, 902, 1102, 1302);
   wherein the aperture stop (603a, 603b, 603c, 803a, 803b, 803c, 803d, 803e, 803f, 1003a, 1003b, 1003c, 1003d, 1003e, 1003f, 1203) of the contact lens is capable of, at least in part, redistributing the incoming light energy entering the model eye into a plurality of oblique spatial frequencies of the spectral signature (702, 902, 1102, 1302);
   wherein the spectral signature (702, 902, 1102, 1302) is different to that obtained when a single vision contact lens with the single vision power profile configured free of the aperture stop (603a, 603b, 603c, 803a, 803b, 803c, 803d, 803e, 803f, 1003a, 1003b, 1003c, 1003d, 1003e, 1003f, 1203) is tested on the model eye under similar conditions; and
   wherein the spectral signature of the retinal image (702, 902, 1102, 1302) mimics a spectral signature obtained by a power spectrum Fourier transform analysis of a natural scene; wherein the natural scene includes a forest scene, a mountain scene, a field scene, a beach scene, a coast scene, a river scene, or a waterfall scene; the contact lens being **characterized in that**
   the transparent region spans an area of at least 12.5 square millimetres; and wherein the non-circular non-transparent region (603, 803, 1003, 1203) spans an area of at least 2.5 square millimetres.

2. The contact lens of claim 1, wherein the non-circular non-transparent region (603, 803, 1003, 1203) is shaped like a regular polygon with more than 3 sides and no greater than 12 sides.

3. The contact lens of anyone of claims 1 to 2, wherein the non-circular non-transparent region (603, 803, 1003, 1203) is not shaped like rectangle, square or a rhombus.

4. The contact lens of anyone of claims 1 to 3, wherein the transparent region includes spherical and/or astigmatic powers.

5. The contact lens of anyone of claims 1 to 4, wherein the transparent region includes positive or negative spherical aberration.

6. The contact lens of anyone of claims 1 to 5, wherein the non-circular non-transparent region (603, 803, 1003, 1203) is translucent, partially opaque, or opaque.

7. The contact lens of anyone of claims 1 to 6, wherein the non-circular non-transparent region (603, 803, 1003, 1203) is decentred with respect to the optical centre of the contact lens.

8. The contact lens of anyone of claims 1 to 7, wherein the non-circular non-transparent region (603, 803, 1003, 1203) is configured such that its diameter is larger than the physiological pupil of the myopic eye, measured in a scotopic or a dim light condition.

9. The contact lens of anyone of claims 1 to 8, wherein the spectral signature of the retinal image (702, 902, 1102, 1302) is different from a spectral signature obtained by a power spectrum Fourier transform analysis of a man-made scene (501, 502); wherein the man-made scene (501, 502) includes an indoor scene, a street scene, a high-building scene, a city-view scene, a highway scene, an office scene, or a portrait scene.

10. The contact lens of anyone of claims 1 to 9, wherein the spectral signature of the retinal image (702, 902, 1102, 1302) provides an optical stop signal to slow the progression of the myopic eye.

11. The contact lens of anyone of claims 1 to 10, wherein the contact lens is capable of providing visible light transmittance that is within at least 80% of the visible light transmittance provided by a conventional single vision contact lens that is free of the non-circular non-transparent aperture stop (603a, 603b, 603c, 803a, 803b, 803c, 803d, 803e, 803f, 1003a, 1003b, 1003c, 1003d, 1003e, 1003f, 1203).

**Patentansprüche**

1. Kontaktlinse für ein kurzsichtiges Auge, wobei die Kontaktlinse eine optische Zone (601, 801, 1001, 1201) mit einem optischen Zentrum umfasst; wobei die optische Zone umfasst:

eine transparente Region mit einem Einstärken-Wirkungsprofil;
eine nicht kreisförmige, nicht transparente Region (603, 803, 1003, 1203), die die transparente Region umschreibt und so konfiguriert ist, dass sie eine Aperturblende (603a, 603b, 603c, 803a, 803b, 803c, 803d, 803e, 803f, 1003a, 1003b, 1003c, 1003d, 1003e, 1003f, 1203) der Kontaktlinse bildet;
wobei die Kontaktlinse, wenn sie durch Testen an einem Modellauge gekennzeichnet ist, das mit einem Entfernungs-Brechungsfehler konfiguriert ist, der mit dem Einstärken-Wirkungsprofil übereinstimmt, ein Netzhautbild (702, 902, 1102, 1302) für mindestens einen Pupillendurchmesser zwischen einschließlich 3 mm und einschließlich 6 mm, für mindestens eine Wellenlänge von einschließlich 420 nm bis einschließlich 760 nm und für mindestens einen weiten Feldwinkel auf der Netzhaut bereitstellt;
wobei das Netzhautbild (702, 902, 1102, 1302), wenn es unter Verwendung einer Leistungsspektrum-Fourier-Transformationsanalyse weiter charakterisiert wird, zu einer spektralen Signatur des Netzhautbildes (702, 902, 1102, 1302) führt;
wobei die Aperturblende (603a, 603b, 603c, 803a, 803b, 803c, 803d, 803e, 803f, 1003a, 1003b, 1003c, 1003d, 1003e, 1003f, 1203) der Kontaktlinse in der Lage ist, zumindest teilweise die eintreffende Lichtenergie, die in das Modellauge eintritt, in einer Vielzahl von schrägen Raumfrequenzen der spektralen Signatur (702, 902, 1102, 1302) umzuverteilen;
wobei die spektrale Signatur (702, 902, 1102, 1302) sich von derjenigen unterscheidet, die erhalten wird, wenn eine Einstärken-Kontaktlinse mit dem Einstärkenprofil, die frei von der Aperturblende (603a, 603b, 603c, 803a, 803b, 803c, 803d, 803e, 803f, 1003a, 1003b, 1003c, 1003d, 1003e, 1003f, 1203) konfiguriert ist, unter ähnlichen Bedingungen an dem Modellauge getestet wird; und
wobei die spektrale Signatur des Netzhautbildes (702, 902, 1102, 1302) eine spektrale Signatur nachahmt, die durch eine Fourier-Transformationsanalyse des Leistungsspektrums einer natürlichen Szene erhalten wird, wobei die natürliche Szene eine Waldszene, eine Bergszene, eine Feldszene, eine Strandszene, eine Küstenszene, eine Flussszene oder eine Wasserfallszene enthält;
wobei die Kontaktlinse **dadurch gekennzeichnet ist, dass**
die transparente Region einen Bereich von mindestens 12,5 Quadratmillimetern überspannt; und wobei die nicht-kreisförmige nicht-transparente Region (603, 803, 1003, 1203) einen Bereich von mindestens 2,5 Quadratmillimetern überspannt.

2. Kontaktlinse nach Anspruch 1, wobei die nicht-kreisförmige, nicht-transparente Region (603, 803, 1003, 1203) die Form eines regelmäßigen Polygons mit mehr als 3 Seiten und nicht mehr als 12 Seiten hat.

3. Kontaktlinse nach einem der Ansprüche 1 bis 2, wobei die nicht-kreisförmige, nicht-transparente Region (603, 803, 1003, 1203) nicht die Form eines Rechtecks, Quadrats oder einer Raute aufweist.

4. Kontaktlinse nach einem der Ansprüche 1 bis 3, wobei die transparente Region sphärische und/oder astigmatische Kräfte enthält.

5. Kontaktlinse nach einem der Ansprüche 1 bis 4, wobei die transparente Region eine positive oder negative sphärische Aberration enthält.

6. Kontaktlinse nach einem der Ansprüche 1 bis 5, wobei die nicht kreisförmige, nicht transparente Region (603, 803, 1003, 1203) durchscheinend, teilweise lichtundurchlässig oder lichtundurchlässig ist.

7. Kontaktlinse nach einem der Ansprüche 1 bis 6, wobei die nicht-kreisförmige, nicht-transparente Region (603, 803, 1003, 1203) in Bezug auf das optische Zentrum der Kontaktlinse dezentriert ist.

8. Kontaktlinse nach einem der Ansprüche 1 bis 7, wobei die nicht kreisförmige, nicht transparente Region (603, 803, 1003, 1203) so konfiguriert ist, dass ihr Durchmesser größer ist als die physiologische Pupille des kurzsichtigen Auges, gemessen unter skotopischer oder schwacher Lichtbedingung.

9. Kontaktlinse nach einem der Ansprüche 1 bis 8, wobei sich die spektrale Signatur des Netzhautbildes (702, 902, 1102, 1302) von einer spektralen Signatur unterscheidet, die durch eine Fourier-Transformationsanalyse des Leistungsspektrums einer vom Menschen geschaffenen Szene (501, 502) erhalten wird; wobei die vom Menschen geschaffene Szene (501, 502) eine Innenraumszene, eine Straßenszene, eine Hochhausszene, eine Stadtansichtszene, eine Autobahnszene, eine Büroszene oder eine Porträtszene enthält.

10. Kontaktlinse nach einem der Ansprüche 1 bis 9, wobei die spektrale Signatur des Netzhautbildes (702, 902, 1102, 1302) ein optisches Stoppsignal bereitstellt, um das Fortschreiten der Kurzsichtigkeit des Auges zu verlangsamen.

11. Kontaktlinse nach einem der Ansprüche 1 bis 10, wobei die Kontaktlinse in der Lage ist, eine Durchlässigkeit für sichtbares Licht bereitzustellen, die innerhalb von mindestens 80 % der Durchlässigkeit für sichtbares Licht liegt, die von einer herkömmlichen Einstärken-Kontaktlinse bereitgestellt wird, die frei von der nicht kreisförmigen, nicht transparenten Aperturblende (603a, 603b, 603c, 803a, 803b, 803c, 803d, 803e, 803f, 1003a, 1003b, 1003c, 1003d, 1003e, 1003f, 1203) ist.

**Revendications**

1. Une lentille de contact pour un œil myope, la lentille de contact comprenant une zone optique (601, 801, 1001, 1201) avec un centre optique ; la zone optique comprenant :

une région transparente avec un profil de correction visuelle unique ;
une région non-circulaire non-transparente (603, 803, 1003, 1203) circonscrivant la région transparente, configurée pour former un arrêt d'ouverture (603a, 603b, 603c, 803a, 803b, 803c, 803d, 803e, 803f, 1003a, 1003b, 1003c, 1003d, 1003e, 1003f, 1203) de la lentille de contact ;

la lentille de contact, lorsqu'elle est **caractérisée par** un test sur un œil modèle configuré avec une erreur de réfraction à distance correspondant au profil de correction visuelle unique, fournit une image rétinienne (702, 902, 1102, 1302) pour au moins un diamètre de pupille compris entre 3 mm et 6 mm inclus, pour au moins une longueur d'onde comprise entre 420 nm et 760 nm inclus, et pour au moins un angle de champ large au niveau de la rétine ;

l'image rétinienne (702, 902, 1102, 1302), lorsqu'elle est en outre **caractérisée par** l'utilisation d'une analyse de transformée de Fourier du spectre de correction, donne une signature spectrale de l'image rétinienne (702, 902, 1102, 1302) ;

l'arrêt d'ouverture (603a, 603b, 603c, 803a, 803b, 803c, 803d, 803e, 803f, 1003a, 1003b, 1003c, 1003d, 1003e, 1003f, 1203) de la lentille de contact est apte, au moins en partie, à redistribuer l'énergie lumineuse entrant dans l'œil modèle en une pluralité de fréquences spatiales obliques de la signature spectrale (702, 902, 1102, 1302) ;

la signature spectrale (702, 902, 1102, 1302) étant différente de celle obtenue lorsqu'une lentille de contact à vision unique ayant le profil de correction visuelle unique configuré de façon à être dépourvu d'arrêt d'ouverture (603a, 603b, 603c, 803a, 803b, 803c, 803d, 803e, 803f, 1003a, 1003b, 1003c, 1003d, 1003e, 1003f, 1203) est testée sur le modèle d'œil dans des conditions similaires ; et

la signature spectrale de l'image rétinienne (702, 902, 1102, 1302) imite une signature spectrale obtenue par une analyse de transformée de Fourier du spectre de correction d'une scène naturelle ; la scène naturelle comprend une scène de forêt, une scène de montagne, une scène de campagne, une scène de plage, une scène de côte, une scène de rivière ou une scène de chute d'eau ;

la lentille de contact étant **caractérisée en ce que**

la région transparente s'étend sur une surface d'au moins 12,5 millimètres carrés ; et la région non-circulaire non-transparente (603, 803, 1003, 1203) s'étend sur une surface d'au moins 2,5 millimètres carrés.

2. La lentille de contact selon la revendication 1, dans laquelle la région non-circulaire non-transparente (603, 803, 1003, 1203) a la forme d'un polygone régulier présentant plus de 3 côtés et pas plus de 12 côtés.

3. La lentille de contact selon l'une quelconque des revendications 1 à 2, dans laquelle la région non-circulaire non-transparente (603, 803, 1003, 1203) n'a pas la forme d'un rectangle, d'un carré ou d'un losange.

4. La lentille de contact selon l'une quelconque des revendications 1 à 3, dans laquelle la région transparente comprend des corrections sphériques et/ou astigmatiques.

5. La lentille de contact selon l'une quelconque des revendications 1 à 4, dans laquelle la région transparente comprend une aberration sphérique positive ou négative.

6. La lentille de contact selon l'une quelconque des revendications 1 à 5, dans laquelle la région non-circulaire non-transparente (603, 803, 1003, 1203) est translucide, partiellement opaque ou opaque.

7. La lentille de contact selon l'une quelconque des revendications 1 à 6, dans laquelle la région non-circulaire non-transparente (603, 803, 1003, 1203) est décentrée par rapport au centre optique de la lentille de contact.

8. La lentille de contact selon l'une quelconque des revendications 1 à 7, dans laquelle la région non-circulaire non-transparente (603, 803, 1003, 1203) est configurée de telle sorte que son diamètre est plus grand que la pupille physiologique de l'œil myope, mesurée dans des conditions scotopiques ou de faible luminosité.

9. La lentille de contact selon l'une quelconque des revendications 1 à 8, dans laquelle la signature spectrale de l'image rétinienne (702, 902, 1102, 1302) est différente d'une signature spectrale obtenue par une analyse de transformée de Fourier du spectre de correction d'une scène artificielle (501, 502) ; dans laquelle la scène artificielle (501, 502) comprend une scène d'intérieur, une scène de rue, une scène de gratte-ciel, une scène de vue sur la ville, une scène d'autoroute, une scène de bureau ou une scène de portrait.

10. La lentille de contact selon l'une quelconque des revendications 1 à 9, dans laquelle la signature spectrale de l'image rétinienne (702, 902, 1102, 1302) fournit un signal d'arrêt optique pour ralentir la progression de l'œil myope.

11. La lentille de contact selon l'une quelconque des revendications 1 à 10, dans laquelle la lentille de contact est apte à fournir une transmittance de la lumière visible qui est comprise dans au moins 80 % de la transmittance de la lumière visible fournie par une lentille de contact à vision unique classique qui est dépourvue dudit arrêt d'ouverture non-circulaire et non-transparent (603a, 603b, 603c, 803a, 803b, 803c, 803d, 803e, 803f, 1003a, 1003b, 1003c, 1003d, 1003e, 1003f, 1203).

Prior art incorporated here in its entirety:
Torralba and Oliva, "Statistics of natural image categories"
Computational Neural Systems, Volume 14 (2003), Page 394.

Figure 1

Prior art incorporated here in its entirety:
Torralba and Oliva, "Statistics of natural image categories"
Computational Neural Systems, Volume 14 (2003), Page 395.

| Natural object | River and waterfall | Forest | Mountain | Field | Beach | Coast |

| Man-made object | Portrait | Indoor scene | Street | High building | City-view | Highway |

Figure 2

EP 4 115 216 B1

Figure 3

Figure 4

501

502

Figure 5

Figure 6

701

702

Fy (c/pixels)

Fx (c/pixels)

Fy (c/pixels)

Fx (c/pixels)

Figure 7

Figure 8

Figure 9

Figure 10

EP 4 115 216 B1

Figure 11

Figure 12

Figure 13

Figure 14

EP 4 115 216 B1

**EP 4 115 216 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2020900607
- GB 1340877 A
- WO 2012102332 A1

**Non-patent literature cited in the description**

- Statistics of natural image categories. Computational Neural Systems. Torralba and Oliva, 2003, vol. 14, 391-412
- **TORRALBA et al.** Statistics of natural image categories. *Computational Neural Systems*, 2003, vol. 14, 394
- **TORRALBA et al.** Statistics of natural image categories. *Computational Neural Systems*, 2003, vol. 14